# EUROPEAN PATENT APPLICATION

(11) **EP 4 711 354 A1**
(43) Date of publication of application: **18.03.2026**
(21) Application number: 24200353.1
(22) Date of filing: 13.09.2024
(51) Int. Cl.: C07C 29/132, C07C 31/20, C07C 29/60

(54) **SHUT-DOWN PROCEDURE FOR A PROCESS FOR THE CONVERSION OF A CARBOHYDRATE SOURCE INTO ALKYLENE GLYCOL**

(71) Applicant: Avantium Knowledge Centre B.V., 1014 BV Amsterdam (NL)
(72) Inventor: VAN BRUMMELEN, Noud, 1014 BV Amsterdam (NL); HOVENGA, Richard, 1014 BV Amsterdam (NL); PUIJK, Matthijs, 1014 BV Amsterdam (NL); RUSSELL, Scott Henry, 1014 BV Amsterdam (NL)
(74) Representative: Avantium Intellectual Property

(57) **Abstract**

A method for shutting-down a process wherein a carbohydrate source is converted into one or more alkylene glycols. In particular the invention relates to a method for shutting- down such a process for the conversion of a saccharide into ethylene glycol. The procedure according to the invention aims to minimize foam formation occurring otherwise.

## Description

### Introduction

The present invention relates to a method for shutting-down a process wherein a carbohydrate source is converted into one or more alkylene glycols. In particular the invention relates to a method for shutting-down such a process for the conversion of a saccharide into ethylene glycol. The procedure according to the invention aims to minimize foam formation occurring otherwise.

### Background of the invention

Alkylene glycols, such as ethylene glycol and propylene glycol, are valuable chemicals. They can be used in the preparation of polyesters, such as polyalkylene terephthalate, in particular polyethylene terephthalate (PET), polyalkylene furanoate (PEF) and polyalkylene naphthenate (PEN). Further applications of alkylene glycols, in particular ethylene glycol, include their use as anti-freeze. These alkylene glycols commonly are produced on a commercial scale by the hydrolysis of alkylene oxides, which are produced from alkylenes and oxygen. The alkylenes are typically products of fossil fuels.

There is a growing interest in the production of alkylene glycols, such as ethylene glycol, from sustainable resources. In US 7,960,594 a process is described wherein ethylene glycol is produced from cellulose. This process involves catalytic degradation and hydrogenation reactions under hydrothermal conditions. More in particular, the process is carried out by contacting cellulose at elevated temperature and pressure with a catalyst system comprising two sorts of active components in the presence of hydrogen. The first active component comprises tungsten or molybdenum in its metallic state or in the form of its carbide, nitride or phosphide. The second component is selected from the hydrogenation metals from Groups 8, 9 and 10 of the Periodic Table of Elements, and includes cobalt, nickel, ruthenium, rhodium, palladium, iridium and platinum. In experiments the catalysts compounds were used on a carrier, such as activated carbon. Moreover, it appears that the reaction conditions that result in satisfactory yields include a temperature of 220 - 250 °C and a hydrogen pressure of 3 to 7 MPa.

This reaction has been further studied for catalyst systems that contain nickel and tungsten on a carrier. There it has been found that nickel and tungsten are leached into the solution during the reaction, which accounts for the gradual deterioration of the catalyst performance (cf. Na Ji et al., ChemSusChem, 2012, 5, 939-944). The leaching of tungsten and other metals has been confirmed in the study reported in M. Zheng et al., Chin. J. Catal., 35 (2014) 602-613. The latter document also discloses that in addition to ethylene glycol different by-products are obtained, including erythritol, glycerol, mannitol and sorbitol.

WO 2016/114661 discloses a continuous process for preparation of ethylene glycol from a carbohydrate source. Said process is carried out in a continuous stirred tank reactor (CSTR) in which a catalyst system is present. Said catalyst system comprises a retro-aldol catalyst comprising a tungsten compound and at least one hydrogenolysis catalyst comprising at least one active metal. The active metal is suitably present in the form of a catalyst supported on a carrier. Such heterogeneous catalyst particles can fairly easily be separated from the effluent stream, e.g. by a sieve plate, and added back. The tungsten compound on the other hand is generally present dissolved or dispersed in the liquid reaction medium (i.e. present as a homogenous catalyst compound) and not so easily removed from the effluent stream. Hence, the tungsten compound is partly removed as part of the effluent in operating the process in a CSTR.

WO2020/055796 describes a shut-down method for a process for the preparation of alkylene glycols from a stream with one or more saccharides in the presence of a catalyst system comprising a retro-aldol tungsten-containing catalyst and a hydrogenation catalyst. The temperature at shut-down may be at least 120°C and the pressure may range from 1MPa to 12 MPa. It states that the catalyst performance may be reduced by deposition of retro-aldol catalyst on any surface. The document observes that the deposition of retro-aldol catalyst is reduced or even eliminated during a shut-down procedure by ensuring that the retro-aldol catalyst is in the presence of an agent suitable to suppress deposition in the reactor when the retro-aldol catalyst is removed from the reactor. Such agents are stated to include: saccharide feed or products formed in the process, such as polyols and glycols, sugar alcohols, ketones or carboxylic acids or sodium salts thereof.

WO2018/044971 in example 1 discloses a process wherein glucose is converted to glycol products with hydrogen in the presence of Raney nickel and sodium metatungstate at a temperature of 230 °C, and a pressure of 1500 psig (about 103 barg) in a continuous stirred tank reactor. After operating for 167.6 hours of operation, the reaction is terminated, and the termination process is described as: the reactor was cooled down and flushed with water to remove glycol products from the spent catalyst, leaving the catalyst as slurry in water. The reactor was de-pressurized, purged with N₂ gas and unbolted.

Applicant has found that when the termination of a carbohydrate conversion is conducted as described in example 1 of WO2018/044971 (when stirring was continued to avoid settling of catalyst particles, which isn't described in this example) substantial foam formation took place in the reactor during the shut-down procedure, which foam ended up in the off-gas treatment equipment. Such foam in the off-gas treatment is undesired as it leads to fouling and safety issues because a lot of hot liquid is in a place that it is not designed for, and thus also economic consequences as systems need to be designed for such, and it also tends to take solids present in the reactor such as hydrogenolysis catalyst particles from the reactor and into the off-gas treatment. This reference in the stated example does not state whether there is or is no stirring during shut down, and it is silent on foam formation.

Therefore, it is desirable to provide a shut-down method for the above-described processes carried out in stirred reactor tanks wherein the risk of foam formation during the shut-down procedure is reduced. Preferably, it is also desired that the amount of undesired products such as degradation products of carbohydrates (e.g. humins) is minimized. The shut-down procedure should be applicable to situations in which the hydrogenolysis catalyst is an active metal selected from the groups 8, 9 and 10 of the Periodic Table of the Elements present in solid form, such as on carrier particulates, e.g. when present as a slurry in the reactor. Preferably, the shut-down process should allow a continuation of stirring of the reactor content during the shut-down procedure so as to prevent heterogeneous catalyst particles form settling.

### Summary of the invention

It was found that the problem may be solved, at least in part, by a method for shutting-down a process, which process prior to shutting down is a process for the production of alkylene glycols in a continuously stirred reactor in which process a carbohydrate source is converted to alkylene glycols with hydrogen in the presence of a retro-aldol catalyst containing a tungsten compound and a hydrogenolysis catalyst comprising an active metal selected from the groups 8, 9 and 10 of the Periodic Table of the Elements at elevated temperature and elevated pressure, which shutting-down method comprises the sequential steps of:
a. terminating liquid flows going in and out of the reactor, and start cooling down of the reactor content,
b. after the reactor content has reached a temperature of below 100°C, replacing at least part of the liquid content of the reactor with water,
c. depressurizing the reactor to atmospheric pressure,
wherein during all of steps a. to c. the reactor content is stirred and during steps a. and b. hydrogen is fed to the reactor.

### Detailed description of the invention

The continuously stirred reactor used for the present process prior to shut-down typically has, under operation, a headspace, e.g. in order to capture excessive hydrogen and volatile side products. Typically, the headspace volume will be between 5 and 60% of the reactor volume, more typically between 10 and 50%.

In the procedure according to the present invention, the reactor content is stirred during all of steps a., b. and c.. This in fact promotes or maintains a tendency to foam formation and is thus disadvantageous on one side, but is deemed necessary to avoid settling of the hydrogenation catalyst, which usually occurs as small particles. Such settled catalyst particles can, when settled, form a cake whilst the reactor is shut-down, thereby hampering emptying the reactor. Stirring can be done by conventional means, such as by an impeller.

In the process of the present invention hydrogen continues to be fed to the reactor at least during steps a. and b. The reason for this is to minimise undesired side reactions (e.g. formation of humins) from occurring. Undesired reactions could take place when catalysts are still present, and hydrogen is not. For this reason, presence of hydrogen is maintained during at least steps a. and b..

As stated above, the reactor content needs to be cooled to a substantial degree before the pressure is released: without cooling to below a substantial point excessive foaming may occur. Also, cooling will terminate reactions from occurring, not only the desired reactions but also undesired reactions. A temperature reduction below 100°C is a practical minimum w.r.t. the amount of temperature reduction that is needed, as reactors at a temperatures above this may require pressurization above atmospheric due to being above the boiling temperature. However, it was found beneficial for both minimizing foam formation as well as ease of handling that cooling is effected to a temperature to below 80°C, and preferably below 60°C. Hence, in the now claimed process it is preferred that in step b. replacing at least part of the liquid content of the reactor with water is commenced after the reactor content has reached a temperature of below 80°C, preferably below 60°C.

Reducing the reactor temperature can be effected by conventional means, e.g. by having a cooling mantle around the reactor or having a heat exchanger present, in which e.g. a cooling liquid circulates.

Next step after ensuring the reactor content has been cooled to a sufficient degree, is replacing at least part of the liquid content of the reactor with water, in step b.. This is done mainly for two reasons: such fresh liquid contains no dissolved hydrogen, so the total amount of dissolved hydrogen and other volatile components is reduced, thereby reducing tendency to foam. Secondly, when the composition of the liquid in the reactor is changed to contain more water and less glycols, foam stability decreases (presumably due to e.g. a change in surface tension) and when foam stability decreases there will be less foam to cause problems in the headspace or downstream part. This replacement of at least part of the liquid with water can be effected by various means. Preferably, in step b. such is effected by replacing at least part of the liquid content of the reactor with water by a flow of water into the reactor and allowing a flow out of the reactor at substantially the same volume as is flowing into the reactor. Having such flow in- and out for e.g. at least half of the mean residence time, preferably linger, e.g. the mean residence time, or twice that, ensures reaction products and also homogeneous catalyst is flushed out. The flow in can be water, or an aqueous liquid.

Although depressurization of the reactor in step c. can occur when the gas in the reactor maintaining the pressure is hydrogen, it is preferred that before such depressurization the hydrogen is replaced, at least in part, with an inert gas. Reason is safety, as then the depressurization has less risk on explosion or fire. Preferably the inert gas for such is selected from the group consisting of nitrogen, helium, neon, argon, krypton, xenon, radon and combinations thereof, the inert gas preferably containing nitrogen, more preferably being nitrogen. Depressurization is typically effected by discharging the gaseous phase (e.g. in the part of equipment that is designed to take the off-gas during operation) until atmospheric pressure is reached in the reactor. However, in an alternative way depressurization is effected by discharging the liquid phase (until atmospheric pressure is achieved in the reactor) in a dedicated storage vessel.

The process that is conducted in the reactor in a continuous way prior to shut-down is as such known in the art, e.g. from WO 2016/114661. More specifically, it is preferred that the process, prior to shut-down, is a process for the production of alkylene glycols is a continuous process wherein a feed stream of said carbohydrate source, a feed stream of the retro-aldol catalyst, and a flow of hydrogen are fed into the reactor in which a hydrogenation catalyst is present and an alkylene glycols-containing product stream is withdrawn from the reactor. In such process the carbohydrate source preferably is selected from the group consisting of cellulose, xylose, starch, glucose, sucrose, fructose, glucose-oligomers, paper waste, and combinations thereof, more preferably glucose, sucrose, starch and combinations thereof, most preferably glucose, sucrose and combinations thereof.

In the stated process in the preceding paragraph the retro-aldol catalyst typically comprises a tungsten compound selected from the group consisting of tungstic acid (H₂WO₄), ammonium tungstate, ammonium metatungstate, ammonium paratungstate, tungstate compounds comprising at least one Group 1 or 2 element, metatungstate compounds comprising at least one Group 1 or 2 element, paratungstate compounds comprising at least one Group 1 or 2 element, tungsten oxide (WO₃), heteropoly compounds of tungsten, and combinations thereof, preferably from the group consisting of tungstic acid, tungstate compounds comprising at least one Group 1 or 2 element and combinations thereof. As to the hydrogenation catalyst in the process as set out above, such preferably comprises an active metal on a carrier, the carrier preferably being selected from carbon, silica, alumina, silica-alumina, zirconia, titania, niobia, iron oxide, tin oxide, zinc oxide, silica-zirconia, zeolitic aluminosilicates, titanosilicates, magnesia, silicon carbide, clays and combinations thereof, and the active metal is preferably selected from the group consisting of Cu, Fe, Ni, Co, Pd, Pt, Ru, Rh, Ir, Os and combinations thereof, preferably selected from the group consisting of Ni, Co, Pd, Pt, Ru and combinations thereof.

Regarding processing conditions for the process according to the present invention, it is preferred that the process for preparing ethylene glycol is conducted at a temperature of 170°C to 350°C, preferably at a temperature of 200°C to 290°C, at a pressure in the range from 1.0 to 16 MPa, preferably from 2.0 to 12 MPa, more preferably from 3.0 to 9.0 MPa.

### EXAMPLES

### Equipment, materials and settings (prior to shut-down)

The hydrogenolysis reactions in the termination experiments were carried out in a continuously stirred tank reactor, of a height : diameter ratio of approximately 1.2.
Introduction of gas was below the impeller.
Headspace volume: about 50% of the total reactor size.
Stirrer speed: 1275 rpm, impeller.

The hydrogenation catalyst used was: ruthenium on carbon particulates. This was a) initially added as a fresh charge and b) during the reaction, fed to the reactor as a slurry in glycerol (approximately 0.2 kg/h), and Ru/C was also continuously removed with the effluent.

Retro-aldol catalyst used was tungstic acid, which was during the process continuous fed to the reactor solubilized in a mixture of ethylene glycol, propylene glycol (together about 50%), water, and NaOH, at a flow rate of 2.8 kg/h.
Carbohydrate feed to reactor was about 31% sucrose in water. Flowrate: 11.5 kg/h.
Reactor temperature during reaction: 230°C.
Pressure in reactor during reaction: 65 bar.
Flowrate of hydrogen during operation: 0.32 kg/h.
Cooling was achieved by external fluid-filled jacket on the reactor wall.

### Experiment 1 (comparative):

After steady state of more than 50 hours the following shut-down sequence was applied:
- the carbohydrate/water feed was switched to just water (7.3 kg/h) (fluid level kept constant), and the catalyst feeds were stopped (total feed, carbohydrate, water, catalyst solutions before shutdown was approx. 14.31 kg/h), and at the same time cooling of the reactor was commenced (stirring and hydrogen flow were continued, though hydrogen was reduced from 0.31 to 0.1 kg/h).

This directly lead to foam formation in the headspace when switching to water even before there was a depressurisation, the foam appearing in the off-gas treatment facility.

### Experiment 2 (comparative):

After steady state of more than 50 hours the following shut-down sequence was applied:
- both the carbohydrate/water feed and the catalyst feeds were stopped (flow out was also stopped, and thus fluid level kept constant), and at the same time cooling of the reactor was commenced (stirring and hydrogen flow were continued, though hydrogen was reduced from 0.32 kg/h to 0.1 kg/h).
- when the reactor contents were at a temperature of 60°C, the reactor was depressurized.

This directly lead to foam formation in the headspace, which foam appeared in the off-gas treatment facility.

### Experiment 3:

After steady state of more than 50 hours the following shut-down sequence was applied:
- both the carbohydrate/water feed and the catalyst feeds were stopped, and at the same time cooling of the reactor was commenced (stirring and hydrogen flow were continued, though hydrogen was reduced from 0.32 kg/h to 0.1 kg/h)
- when the reactor contents were at a temperature of 60°C, water was pumped into the reactor, in a total volume of 2x the reactor volume. The level of the fluid in the reactor was kept about constant by allowing the same flow out of the reactor content as was the flow into with water (stirring and hydrogen was replaced with nitrogen at 0.1 kg/h).
- the reactor was depressurized to atmospheric pressure (stirring was continued).

This lead to a shut-down without excessive foam formation.

## Claims

1. A method for shutting-down a process, which process prior to shutting down is a process for the production of alkylene glycols in a continuously stirred reactor, in which process a carbohydrate source is converted to alkylene glycols with hydrogen in the presence of a retro-aldol catalyst containing a tungsten compound and a hydrogenolysis catalyst comprising an active metal selected from the groups 8, 9 and 10 of the Periodic Table of the Elements at elevated temperature and elevated pressure, which shutting-down method comprises the sequential steps of:
a. terminating liquid flows going in and out of the reactor, and start cooling down of the reactor content,
b. after the reactor content has reached a temperature of below 100°C, replacing at least part of the liquid content of the reactor with water,
c. depressurizing the reactor to atmospheric pressure,
wherein during all of steps a. to c. the reactor content is stirred and during steps a. and b. hydrogen is fed to the reactor.

2. Method according to claim 1, wherein in step b. replacing at least part of the liquid content of the reactor with water is commenced after the reactor content has reached a temperature of below 80°C, preferably below 60°C.

3. Method according to claim 1 or 2, wherein in step b. replacing at least part of the liquid content of the reactor with water is effected by a flow of water into the reactor and allowing a flow out of the reactor at substantially the same volume as is flowing into the reactor.

4. Method according to any of the preceding claims, wherein during or after step b. the hydrogen is replaced, at least in part, with an inert gas.

5. Method according to claim 4, wherein the inert gas is selected from the group consisting of nitrogen, helium, neon, argon, krypton, xenon, radon and combinations thereof, the inert gas preferably containing nitrogen, more preferably being nitrogen.

6. Method according to any one of the preceding claims, wherein the process for the production of alkylene glycols is a continuous process wherein a feed stream of said carbohydrate source, a feed stream of the retro-aldol catalyst, and a flow of hydrogen are fed into the reactor in which a hydrogenation catalyst is present and an alkylene glycols-containing product stream is withdrawn from the reactor.

7. Method according to any one of the preceding claims, wherein the carbohydrate source is selected from the group consisting of cellulose, xylose, starch, glucose, sucrose, fructose, glucose-oligomers, paper waste, and combinations thereof, more preferably glucose, sucrose, starch and combinations thereof, most preferably glucose, sucrose and combinations thereof.

8. Method according to any of the preceding claims, wherein the retro-aldol catalyst comprises a tungsten compound selected from the group consisting of tungstic acid (H₂WO₄), ammonium tungstate, ammonium metatungstate, ammonium paratungstate, tungstate compounds comprising at least one Group 1 or 2 element, metatungstate compounds comprising at least one Group 1 or 2 element, paratungstate compounds comprising at least one Group 1 or 2 element, tungsten oxide (WO₃), heteropoly compounds of tungsten, and combinations thereof, preferably from the group consisting of tungstic acid, tungstate compounds comprising at least one Group 1 or 2 element and combinations thereof.

9. Method according to any of the preceding claims, wherein the hydrogenation catalyst comprises an active metal on a carrier, the carrier preferably being selected from carbon, silica, alumina, silica-alumina, zirconia, titania, niobia, iron oxide, tin oxide, zinc oxide, silica-zirconia, zeolitic aluminosilicates, titanosilicates, magnesia, silicon carbide, clays and combinations thereof, and the active metal is preferably selected from the group consisting of Cu, Fe, Ni, Co, Pd, Pt, Ru, Rh, Ir, Os and combinations thereof, preferably selected from the group consisting of Ni, Co, Pd, Pt, Ru and combinations thereof.

10. Method according to any of the preceding claims, wherein the process for preparing ethylene glycol is conducted at a temperature of 170°C to 350°C, preferably at a temperature of 200°C to 290°C, at a pressure in the range from 1.0 to 16 MPa, preferably from 2.0 to 12 MPa, more preferably from 3.0 to 9.0 MPa.
